# DEMANDE DE BREVET EUROPEEN

(11) **EP 1 659 404 A1**
(43) Date de publication de la demande: **24.05.2006**
(21) Numéro de dépôt: 04405710.7
(22) Date de dépôt: 17.11.2004
(51) Int. Cl.: G01N 33/543, G01N 33/569

(54) **Dispositif de diagnostic autonome miniaturise**

(71) Demandeur: Valtronic S.A., 1343 Les Charbonnières (CH)
(72) Inventeur: Rochat, Michel, 1167 Lussy s/Morges (CH); Lemaire, Lionel, 1168 Villars sous Yens (CH)
(74) Mandataire: GLN

(57) **Abrégé**

L'invention concerne un dispositif de diagnostic pour la détection d'un agent infectieux dans le sang. Il comporte, réunis sur un support miniaturisé (10):
- un détecteur (16) apte à recevoir un échantillon sanguin, comprenant un récepteur biologique destiné à fixer l'agent infectieux contenu dans l'échantillon, et dont une propriété physique est modifiée par la fixation de l'agent sur le récepteur,
- un circuit de traitement (20) couplé au détecteur et ayant pour fonction de fournir une information représentative de la propriété physique, et
- des moyens d'affichage (24) de cette information.

## Description

La présente invention se rapporte au domaine biomédical. Elle concerne, plus particulièrement, un dispositif miniaturisé permettant de réaliser des diagnostics médicaux de manière autonome.

La détection de maladies infectieuses et, plus particulièrement des virus HIV, HCV et CMV, représente une grande partie des tests sanguins effectués. Une telle détection consiste à doser la charge virale ou en cellules immunitaires contenue dans le sang. Elle permet, d'une part, d'établir un diagnostic et, d'autre part, d'effectuer un suivi de l'état d'un patient afin d'adapter régulièrement son traitement.

A l'heure actuelle, le dosage de la charge virale requiert un site d'intervention complètement équipé, ainsi que du personnel qualifié, afin de collecter les échantillons et de les envoyer dans des laboratoires sophistiqués. Les tests effectués sont complexes. Ils consistent généralement en une suite d'opérations visant à séparer le plasma des cellules sanguines, extraire l'ADN/ARN du virus ou de la bactérie par destruction de sa membrane, amplifier les séquences ADN/ARN caractéristiques de l'agent infectieux et, enfin, détecter ces séquences caractéristiques à l'aide de marqueurs, souvent par une méthode optique. En raison du coût élevé et de la centralisation de ces analyses, les populations les plus démunies ou vivant dans des régions reculées, n'y ont que peu accès.

Dans le cas du SIDA, le meilleur indicateur de niveau de destruction du système immunitaire par le HIV et des risques de progression clinique de la maladie est le nombre des anticorps CD4 et la charge virale. Il est donc important, d'une part, d'effectuer un diagnostic au plus tôt après une exposition au virus et, d'autre part, de contrôler périodiquement la charge virale d'un patient afin de réagir rapidement à l'évolution de la maladie.

L'un des buts de la présente invention est de fournir un dispositif relativement bon marché, capable d'effectuer de telles analyses de manière complètement autonome, en un lieu quelconque et sans personnel qualifié, afin d'en généraliser l'utilisation.

De façon plus précise, l'invention concerne un dispositif de diagnostic pour la détection d'un agent infectieux dans le sang, caractérisé en ce qu'il comporte, réunis sur un support miniaturisé :
- un détecteur constitué d'un microsystème électromécanique apte à recevoir un échantillon sanguin, comprenant un récepteur biologique destiné à fixer l'agent infectieux contenu dans l'échantillon, et dont une propriété physique est modifiée par la fixation de l'agent sur le récepteur,
- un circuit de traitement couplé au détecteur et ayant pour fonction de fournir une information représentative de la propriété physique, et
- des moyens d'affichage de cette information.

Le dispositif de diagnostic selon l'invention comporte encore les principales caractéristiques suivantes :
- Le détecteur est constitué d'un microsystème électromécanique.
- Il comporte, en outre, monté sur le support en amont du détecteur, un microsystème électromécanique destiné au prélèvement de l'échantillon sanguin et à sa filtration en vue d'éliminer les cellules sanguines pour ne conserver que le plasma.
- Le détecteur et le microsystème de prélèvement appartiennent à un module détachable du support afin de permettre son interchangeabilité.

D'autres caractéristiques apparaîtront plus clairement à la lecture de la description qui suit, faite en référence aux dessins annexés, dans lesquels :
- la figure 1 est un schéma général du dispositif selon l'invention, et
- la figure 2 est une vue agrandie de son système de prélèvement.

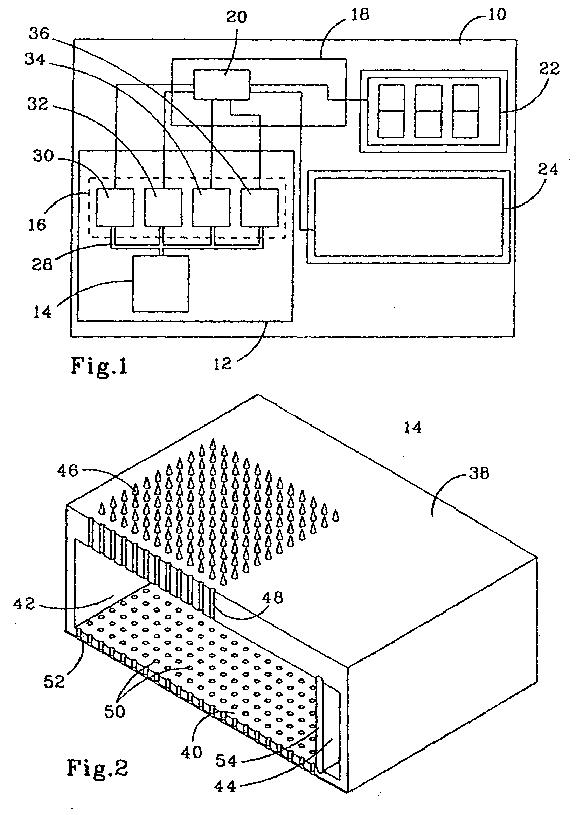

La figure 1 montre un dispositif de diagnostic selon l'invention pour la détection d'un agent infectieux dans du sang. Il comprend, schématiquement, disposés sur un circuit imprimé 10 doté de connexions électriques et fluidiques :
- un module de test 12 comportant un bloc de prélèvement et de filtration de sang 14 et un bloc détecteur 16,
- un module de contrôle 18 comportant un microprocesseur 20,
- un module d'affichage 22 pour signifier le résultat du test, et
- un module d'alimentation en énergie 24 de l'ensemble.

Le dispositif selon l'invention a, environ, la taille d'une carte de crédit et une épaisseur de l'ordre de deux à cinq millimètres.

Le bloc 14 est constitué d'un microsystème électromécanique qui sera décrit ultérieurement en regard de la figure 2. Sa fonction est de prélever un petit volume de sang de l'ordre de cent micro-litres, et de le filtrer afin de séparer le plasma et les virus des cellules sanguines, en vue d'éliminer ces dernières.

Après cette opération, le volume de liquide restant est pratiquement diminué d'un facteur deux. La concentration du virus dans le plasma est alors sensiblement deux fois plus élevée que dans le sang, ce qui a pour effet d'augmenter d'autant la sensibilité de l'analyse ultérieure.

Le bloc 14 est relié au bloc détecteur 16 par l'intermédiaire de connexions fluidiques 28 qui permettent de lui amener le liquide filtré. Ces connexions sont, par exemple, des canaux gravés dans le support du circuit imprimé 10.

Dans une configuration particulièrement intégrée, les blocs 14 et 16 peuvent être construits au sein du même substrat.

Le bloc détecteur 16 est constitué, par exemple, de quatre microsystèmes électromécaniques 30, 32, 34 et 36 disposés en parallèle et simultanément capables, chacun, de détecter un agent particulier, tel que les virus HIV1 et HIV2, ou des anticorps de type CD4. Ces microsystèmes sont fabriqués, par exemple, par la technologie des MEMS (de l'anglais Micro-Electronic Mechanical System), c'est-à-dire par micro-usinage de plaquettes de silicium ou de verre.

La mise en évidence d'un agent infectieux se décompose généralement en deux étapes, soit l'extraction d'ADN/ARN, puis la détection. Dans certains cas, l'étape d'extraction n'est pas nécessaire pour effectuer un diagnostic et l'agent infectieux est mis en évidence par une méthode directe. Le virus est alors détecté par l'intermédiaire de protéines situées sur son enveloppe.

L'extraction d'ADN/ARN, encore appelée lyse, peut être réalisée, à l'échelle macroscopique, par différentes techniques. L'une d'elles, consistant à mélanger le plasma avec un réactif, puis à chauffer le tout à une température contrôlée, a été adaptée avec succès à l'échelle microscopique. Une description complète de ce procédé est donnée dans l'article de Burns et al. intitulé 'An integrated nanoliter DNA analysis device' (Science 282, 1998, p484). L'extracteur miniaturisé associé fait partie intégrante d'un microsystème électromécanique capable d'effectuer une analyse complète, depuis le dosage d'un échantillon sanguin, jusqu'à la détection par une méthode optique.

Si la technique de détection le requiert, les microsystèmes 30, 32, 34 et 36 du bloc détecteur 16 intègrent l'extracteur d'ADN/ARN miniaturisé mentionné ci-dessus. Bien entendu, toute autre technique d'extraction à l'échelle microscopique peut être utilisée.

Dans le domaine de l'analyse biomédicale, les méthodes traditionnelles de détection d'un agent infectieux, par électrophorèse ou par fluorescence, utilisent généralement sa séquence ADN/ARN caractéristique. Elles nécessitent cependant une source ainsi qu'une détection optiques, et ne sont, par conséquent, pas adaptées à un dispositif autonome intégré.

De nouvelles méthodes purement électriques, développées ces dernières années, trouvent, par contre, une application intéressante dans le dispositif selon l'invention. Toutes font appel à un détecteur comportant un agent biologique récepteur sur lequel vient se fixer l'agent infectieux lui-même, ou sa séquence ADN/ARN caractéristique, ce qui a pour effet de modifier une propriété électrique du détecteur.

L'une de ces méthodes, décrite dans l'article de Terrettaz et al. intitulé 'Immunosensing by a synthetic ligand-gated ion channel' (Angew. Chem. Int. Ed. 2001, 40, N°9, p1740), utilise un détecteur constitué d'une électrode en or recouverte d'une double couche de lipides isolante, elle même recouverte d'une couche de protéines réceptrices, le tout étant immergé dans une solution conductrice. La fixation de l'agent infectieux, par l'intermédiaire d'une protéine située sur son enveloppe, ou d'une séquence ADN/ARN complémentaire à la protéine réceptrice, induit une modification de l'impédance de la couche de lipides, mesurable électriquement.

Une autre technique, présentée dans un article de llic et al. intitulé 'Mechanical resonant immunospecific biological detector' (App. Phys. Lett. 77, N°3, 2000) permet de déceler la présence de bactéries ou de virus à l'aide d'un détecteur constitué d'un oscillateur micro-mécanique comportant, à sa surface, une protéine réceptrice. La fixation d'un agent infectieux ou d'une séquence ADN/ARN, à la protéine modifie la fréquence de résonance de l'oscillateur et, par là même, permet sa détection.

Les deux techniques de détection mentionnées ci-dessus, qui utilisent des détecteurs de type électrique, sont parfaitement compatibles avec un dispositif entièrement autonome et de taille réduite. L'une ou l'autre d'entre elles, de même que toute autre technique électrique se prêtant à une application à l'échelle microscopique, peut être implantée sur les microsystèmes 30, 32, 34 ou 36, selon tout procédé à disposition de l'homme de métier. Les composants biologiques nécessaires à l'analyse, tels que les lipides ou les protéines, sont intégrés aux microsystèmes lors de leur fabrication. L'opération d'extraction préalable est également, ou non, réalisée par les mêmes microsystèmes, si la technique de détection le requiert ou non.

Sur une deuxième zone du circuit, se trouve le module de contrôle 18 du dispositif autonome selon l'invention. Ce module est un circuit de traitement constitué d'un microprocesseur 20 ainsi que de toute l'électronique nécessaire à son fonctionnement, relié par des pistes électriques aux modules, respectivement, de test 12, d'affichage 22 et d'alimentation 24.

Le rôle du module 18 est de gérer l'ensemble des opérations du dispositif, et en particulier, les opérations de test, de la purification du sang à la détection de l'agent infectieux. L'information électrique, délivrée par les différents microsystèmes du bloc d'analyse 16 à l'issue de l'étape de détection, est sous forme d'une mesure d'impédance ou de fréquence d'oscillation. Cette information est traitée par le circuit de traitement qui en extrait une indication de la présence de l'agent infectieux recherché, de type qualitatif ou quantitatif.

L'indication délivrée par le circuit de traitement est, ensuite, affichée par l'intermédiaire du module d'affichage 22 qui occupe une troisième zone sur le dispositif. Une autre possibilité serait de transmettre cette information, par une connexion sans fil, à un système extérieur de type montre ou terminal, portable ou non.

Enfin, le module d'alimentation 24, occupant une quatrième zone sur le dispositif, consiste, par exemple, en une batterie ou une cellule photovoltaïque.

La figure 2 est une vue agrandie du bloc de prélèvement et de filtration 14, destiné à prélever et filtrer un petit volume de sang. Ce bloc est constitué d'un microsystème électromécanique ayant la forme d'un parallélépipède creux, en silicium ou verre, dont le volume intérieur, délimité par une paroi supérieure 38, une paroi inférieure 40 et quatre parois latérales, forme un réservoir 42 et une chambre 44 attenante, entièrement fermée, dont la fonction sera explicitée ultérieurement.

La paroi supérieure est munie d'un réseau de micro-aiguilles 46 permettant de prélever de manière indolore des micro-doses de sang à travers le derme d'un doigt pressé contre elles. Ces micro-aiguilles sont obtenues, par exemple, par la technologie des MEMS. Typiquement, les aiguilles ont des dimensions de 50 à 80 microns de large, 100 à 150 microns de hauteur et un espacement d'environ 200 microns. A leur base, un canal 48 percé à travers la paroi 38 permet au sang de s'écouler dans le réservoir 42.

La paroi inférieure 40 est munie d'un réseau de micro-trous 50 de diamètre de l'ordre de 5 à 50 microns, destiné à filtrer grossièrement le sang. Elle constitue le support d'une membrane 52, déposée du côté extérieur de la paroi 40, et formée d'un film poreux tel qu'une céramique (par exemple, l'oxyde d'aluminium) ou un polymère. Cette membrane 52 permet, en sortie du filtre grossier, de filtrer finement le sang. En effet, les cellules sanguines, dont les dimensions vont du micron à la dizaine de microns, sont retenues par la membrane 52, tandis que les anticorps et les virus contenus dans le plasma, et dont les dimensions sont de l'ordre de la dizaine à la centaine de nanomètres, la traversent.

Une surpression dans le réservoir 42 peut être nécessaire pour forcer le passage du plasma sanguin à travers la membrane 52 et le pousser jusqu'au bloc détecteur 16 par l'intermédiaire des connexions 28. A cet effet, la chambre 44 contenant de l'air est séparée du réservoir 42 contenant du sang par une membrane élastique 54, par exemple en silicium. Une résistance chauffante située à l'intérieur ou à l'extérieur de la chambre 44 permet de dilater l'air qui y est contenu et de provoquer ainsi une déformation de la membrane 54. Celle-ci exerce alors une surpression dans le réservoir 42. Afin d'éviter un reflux du sang dans les canaux 48, un système d'anti-retour, non représenté sur la figure 2, est disposé à la base des micro-aiguilles. Une autre possibilité pour faire passer le sang à travers la membrane de filtration 52 est d'utiliser une micro-pompe telle que décrite dans l'article de K. Handique et al. intitulé 'On-chip thermopneumatic pressure for discrete drop pumping' (Analytical Chemistry, Vol 73, N°8, April 15, 2001).

Dans la description du dispositif de diagnostic représenté sur la figure 1, le microsystème 14 possède les fonctions de prélèvement et de filtration, tandis que les microsystèmes 30, 32, 34, 36 possèdent chacun au moins la fonction de détection, et éventuellement, celle d'extraction. Il serait cependant parfaitement envisageable de réunir sur un même microsystème électromécanique les fonctions de prélèvement, filtration et extraction, ou même toutes les fonctions. Inversement, il serait possible de séparer physiquement les quatre différentes fonctions, chaque fonction correspondant à un microsystème différent. Toutes les combinaisons de fonctions, pour autant qu'elles respectent l'ordre du protocole, peuvent donner lieu à des réalisations légèrement différentes, au sein du module de test.

Le module de test 12 est, par mesure d'hygiène et du fait de l'utilisation de réactifs et composants biologiques par les microsystèmes 30, 32, 34 et 36, à usage unique. Les modules de contrôle 18, d'affichage 22 et d'alimentation 24, sont, en revanche, réutilisables. Sur une variante particulièrement avantageuse du dispositif 10, le module de test 12 forme une sorte de cassette détachable et jetable.

## Revendications

1. Dispositif de diagnostic pour la détection d'un agent infectieux dans le sang, **caractérisé en ce qu'**il comporte, réunis sur un support miniaturisé (10):
- un détecteur (16) apte à recevoir un échantillon sanguin, comprenant un récepteur biologique destiné à fixer l'agent infectieux contenu dans ledit échantillon, et dont une propriété physique est modifiée par la fixation dudit agent sur ledit récepteur,
- un circuit de traitement (20) couplé audit détecteur et ayant pour fonction de fournir une information représentative de ladite propriété physique, et
- des moyens d'affichage (22) de cette information.

2. Dispositif selon la revendication 1, **caractérisé en ce que** ladite propriété physique est son impédance électrique.

3. Dispositif selon la revendication 1, **caractérisé en ce que** ladite propriété physique est sa fréquence de résonance.

4. Dispositif selon l'une des revendications 1 à 3, **caractérisé en ce** ledit détecteur (16) est constitué d'un microsystème électromécanique (30, 32, 34, 36).

5. Dispositif selon l'une des revendications 1 à 4, **caractérisé en ce qu'**il comporte, en outre, monté sur ledit support (10) en amont dudit détecteur (16), un microsystème électromécanique (14) destiné au prélèvement de l'échantillon sanguin et à sa filtration en vue d'éliminer les cellules sanguines pour ne conserver que le plasma.

6. Dispositif selon la revendication 5, **caractérisé en ce que** ledit microsystème (14) comporte un réservoir (42) :
- dont une première paroi (38) est munie d'un réseau de micro-aiguilles (46) destinées à percer le derme d'un doigt pressé contre elles afin d'en extraire du sang et de le recueillir dans ledit réservoir (42),
- dont une deuxième paroi (40) possède une structure assurant ladite fonction de filtration, et
- dont une troisième paroi est constituée d'une membrane élastique (54) déformable de manière à mettre le contenu du réservoir (42) sous pression et forcer ainsi son passage à travers ladite deuxième paroi pour l'envoyer audit détecteur.

7. Dispositif selon la revendication 6, **caractérisé en ce que** ladite membrane élastique (54) est associée à une résistance électrique destinée à provoquer sa déformation.

8. Dispositif selon l'une des revendications 6 et 7, **caractérisé en ce que** ladite deuxième paroi (40) est munie d'un réseau de micro-trous (50) et supporte une membrane poreuse (52).

9. Dispositif selon l'une des revendications 5 à 8, **caractérisé en ce que** ledit détecteur (16) et ledit microsystème de prélèvement (14) appartiennent à un module détachable dudit support (10) afin de permettre son interchangeabilité.

10. Dispositif selon l'une des revendications 1 à 9, **caractérisé en ce qu'**il comporte, en outre, un extracteur (16) monté sur ledit support (10), entre ledit détecteur (16) et le microsystème (14) de prélèvement, et destiné à l'extraction d'ADN/ARN.

11. Dispositif selon les revendications 9 et 10, **caractérisé en ce que** ledit extracteur (16) appartient également audit module détachable du support (10).

12. Dispositif selon l'une des revendications 10 et 11, **caractérisé en ce que** ledit extracteur (16) et ledit détecteur (16) forment un même microsystème électromécanique (30, 32, 34, 36).

13. Dispositif selon l'une des revendications 1 à 12, **caractérisé en ce qu'**il comporte, en outre, sur ledit support miniaturisé (10), un module d'alimentation (24) électrique des différents composants.

14. Dispositif selon l'une des revendications 4 à 13, **caractérisé en ce que** ledit détecteur (16) est constitué d'une pluralité de microsytèmes (30, 32, 34, 36) destinés à détecter chacun un agent infectieux.
